# EUROPEAN PATENT SPECIFICATION

(11) **EP 2 804 662 B1**
(45) Date of publication and mention of the grant of the patent: **30.03.2016**
(21) Application number: 13705904.4
(22) Date of filing: 15.01.2013
(51) Int. Cl.: A61N 1/04

(54) **A DEVICE FOR TREATMENT OF A WOUND**
VORRICHTUNG ZUR BEHANDLUNG EINER WUNDE
DISPOSITIF POUR LE TRAITEMENT D'UNE PLAIE

(30) Priority: 16.01.2012 DK 201200040
(43) Date of publication of application: 26.11.2014
(73) Proprietor: Texo Medical ApS, 2000 Frederiksberg (DK)
(72) Inventor: BUSTED, Tommy, DK-2300 Copenhagen S (DK)
(74) Representative: Høiberg A/S
(86) International application number: PCT/DK2013/050012
(87) International publication number: WO 2013/107458

(56) References cited:
- CA-A1- 2 557 687
- US-A1- 2004 267 169
- US-A1- 2005 085 751
- US-A1- 2005 187 580
- US-A1- 2007 276 318
- US-A1- 2008 103 550
- US-A1- 2009 143 761
- US-A1- 2009 149 800
- US-A1- 2011 118 655
- US-B1- 6 413 255

## Description

The present disclosure relates to the field of wound dressings and relates to a device for treatment of a wound by attachment to the skin, comprising a set of electrodes arranged on a carrier member and connected to an electrical power source having a first and a second terminal which are connected to each of the electrodes.

Wound healing typically takes place in three different phases. Even though these phases intertwine and overlap, each has a specific sequence of events which separates them.

During the initial or inflammatory phase the body begins to cleanse away bacteria and begin haemostasis. The inflammatory phase has three subphases: haemostasis, leukocyt and macromigration and epithelialisation. This phase typically takes about four days.

The second phase, the proliferative phase, is characterised by a spreading of fibroblasts, collagen synthesis, granulation and wound contraction. The proliferative phase typically begins about 48 hours after the inflection of the wound and can stretch from anything between two hours and up to a week. In this phase the fibroblast cell synthesis begins and also deposition of the protein collagen which will make up the most important structural matrix for a successful healing of the wound.

In the third phase, remodelling, collagen slows down production. The collagen formed in this phase is more well organized than collagen formed during the proliferative phase. Lastly the new collagen increases the tensile strength of the wound to about 80% of the original strength of the skin.

This is the general process that takes place in healthy people. Patients suffering from diseases that reduce the flow of blood to the wound are unfortunately not able to perform the normal wound healing process as described. In some patients, this process has stopped. Factors that can have a negative influence on the normal wound healing process comprise diabetes, reduced mobility, infection, malnutrition, and medicine. Other factors such as traumatic lesion and burns can also damage the natural wound healing process.

For various reasons, bad blood circulation is the primary cause of chronic wounds, such as venous wounds, diabetic wounds, and decubitus foot ulcers. Venostasis wounds are typically just above the patient's ankles. The blood circulation in this region of the legs of older people or patients incapacitated from employment can be very poor which leads to dehydration of skin cells. These skin cells are thus oxygen starved and poisoned by their own waste products and begin to die. When they are dead, they leave an open leg wound which has a very poor chance of healing itself. Diabetic foot wounds around the ankle and foot also have very poor conditions for healing due to the very poor blood circulation.

Decubitus ulcers are when the skin is exposed to constant pressure and movement to ensure blood circulation is not possible. The lack of blood circulation leads to the same degenerative process as described above, paraplegics and extremely immobile older patients lacking the ability to turn over in bed are the most important candidates to this problem.

In the art of wound treatment various forms of dressings and plasters are known which, possibly combined with antibiotics and treatments with cleansing chemicals, are to protect and isolate the wound so that healing can take place. However, these techniques are often insufficient in case of complicated lesions as described above, where the body is not able to develop a sufficient regeneration ability.

A method of stimulating healing of wounds is known, among others, from US 2008/103550 and WO02/098502. This and other patents are based on a method for improving wound healing by using electric stimulation of the wound by means of a device, e.g. in form of a battery supplying voltage and thereby power surrounding and penetrating the wound by having two electrodes of e.g. thin metal connected to a power source which is powerful enough to generate a current between the electrodes and thereby the wound. US2005/085751 describes how a conductive material is placed between the two electrodes, e.g. in form of hydrogel, this material can furthermore contain silver in some form.

There is a need for changing and improving the above-mentioned techniques to make the healing process even more effective. There is a growing need for treatment of complicated wounds which are a burden not only to the patients but also the healthcare staff, which must allocate many resources for the treatment of wounds. It would also be desirable to have treatments that could reduce the consumption of antibiotics.

Accordingly, the invention consists in a device for treatment of a wound by attachment to the skin, comprising a set of electrodes arranged on a carrier member and connected to an electrical power source having a first and a second terminal which are connected to each of the electrodes, wherein the set of electrodes comprises two non-interconnected electrically conducting wires arranged in a pattern on said carrier member and that the electrically conducting wires are provided with an irregular surface structure.

Hereby, a device in the form of a plaster or similar wound dressing is provided with an electrical stress field is created that affects the wound and the surrounding tissue. The power supply, i.e. the electrical power source, is preferably a remotely stored battery or the like supplying an electrical current to the wires in plaster, i.e. the carrier member of the device. The wires are pre-treated to create an irregular stress field above the damaged tissue in the area of the wound. This electrical stimulation of the damaged tissue results in a treatment which sends non-linear electrical signals through the tissue and among other things hit arteries, arterioles, capillaries, venules and veins

At the capillaries, which are the smallest blood vessels, the exchange of gasses and nutrients and waste matters between the blood and tissue cells takes place. Arteries and arterioles (mentioned according to decreasing size) transport blood from the heart to the capillaries. Venules and veins (mentioned according to increasing size) return the blood to the heart. When the flow hits these parts, the hormone-like substances nitrogen oxide (NO) and prostacyclin (prostaglandin I₂, PGl₂) are provoked, among other things. NO is inorganic matter which inhibits the adhesion of the thrombocytes. Both are generated in and released from endothelial cells, both cooperate to inhibit the activation of the thrombocytes, and both are relaxing to the musculature of the vessels (vasodilating). This causes blood to flow to the area of the wound and prevents the blood vessels to clog. Hereby, the device according to the invention is advantageous since the device facilitates the healing of the wound.

Preferably, the conducting wires are made of a precious metal, such as gold, silver or platinum. Hereby, a superior electric conductivity is achieved. It is further found advantageous that the conducting wires are between 0.15-0.40 mm in diameter.

The set of non-interconnected conducting wires are in an aspect substantially parallelly arranged in a serpentine-like pattern. However, other patterns in particular irregular patterns may be provided in order to obtain an irregular electrical field and thereby impacting a larger amount of tissue in the area of the wound.

The carrier member has a distal side and a proximal side, and preferably the set of conducting wires are adhesively attached in the pattern to the proximal side of the carrier member and with a cover sheet. Moreover, the conducting wires are arranged to penetrate through the carrier member where the wires are connected on the distal side of the carrier member to a cable from the power supply. This arrangement ensures a device which is easy to apply and to wear and directs the electrical field towards the wound area covered by the device. Said connection between the wires and the cable is preferably covered with an electrically shielding sheet material and a covering top sheet thereon. This shielding material is preferably polytetrafluoroethylene (PFTE) (i.e. Teflon®) or the like which is electrically insulating.

In an aspect at least one of the wires is connected to the cable via a resistor, preferably having an electrical resistance of approx. 1 kΩ. Hereby, when moisture in the covered area of the damaged tissue increases the resistors will ensure a voltage drop so that the electrical current in the wires is turned down. Hereby, the wearer will not experience any discomfort with regard to the electrical stimulations of the device.

In a device according to the invention, the electrical power source preferably provides a direct current, preferably with a voltage between 3.5-20 V with a current preferably less than 400 mA. Hereby, the wearer will not experience any discomfort when having the device attached and the electrical power provided is nevertheless found sufficient for ensuring the electrical stimulation of the damaged tissue and thereby facilitate healing a wound.

The pre-treatment may consist in the conductive material being adapted so that the physical structure of the conductor is altered in several random places on the conductor, for example by a mechanical treatment of the conductor so the electrons running in the conductor meet areas of variable electric properties.

The pretreatment can also consist in a chemical treatment of the conductor whereby material can both be removed and another conductive material can be added.

The conductors which are not connected are laid in different patterns to further create interference in the stress field between the conductors.

Alternatively, one of the conductors has, via an electrolytic treatment, been corroded in the surface so that it is capable of possibly containing/absorbing a medicine which is added to the precious metal for being deposited in the tissue.

The invention is explained in more detail with reference to the drawings, in which:
- Fig. 1: is a top view of a wound plaster of a device according to an aspect;
- Fig. 2: is the same the same with indications of hidden details therein;
- Fig. 3: is a schematic cross-sectional view of the plaster of the device shown in figures 1 and 2;
- Fig. 4: is a schematic exploded view of the plaster of the device shown in the previous figures; and
- Figs. 5 a) and b): are two schematic views of examples of pre-treated conductive wires of a device.

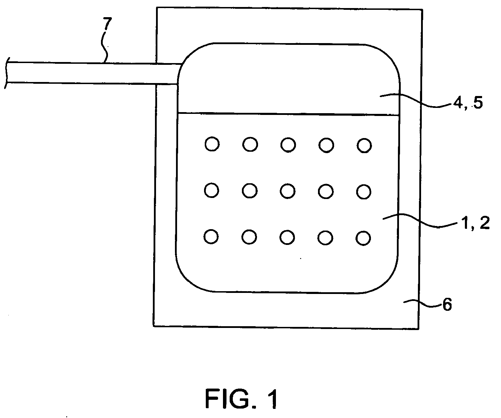

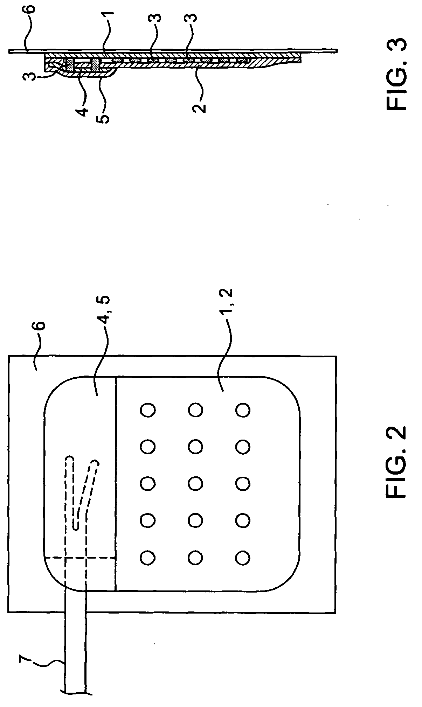

With reference to figures 1-4, the device comprises a plaster which is connectable to an electrical power source. The plaster is made of a base layer 1 and a carrier member 2. A set of conductor wires 3 is arranged between the carrier member 2 and the base layer 1. Preferably the conductor wires 3 are adhesively attached to the proximal side of the carrier member 2, i.e. the side facing towards the base layer 1, and the carrier member 2 is adhered to the top of the base layer 1, whereby the conductor wires 3 are "sandwiched" between the base layer 1 and the carrier sheet 2.

The base layer is provided with a skin-friendly adhesive on its other side, i.e. the lower of proximal side of the base layer 1. This is to attach the plaster to the skin of a patient. A protective release liner 6 is provided to protect the adhesive layer of the plaster during storage and is removable before use of the plaster.

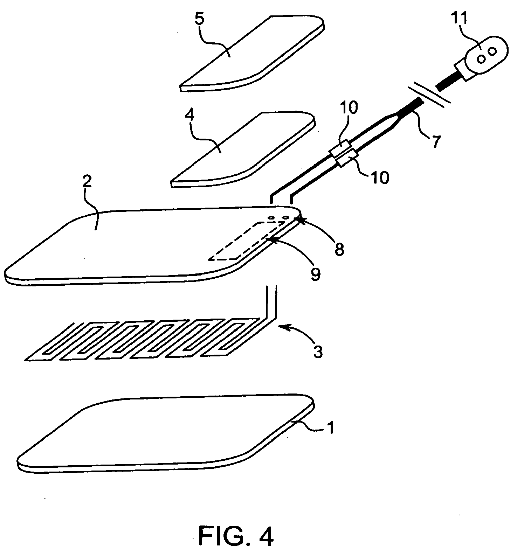

The conductive wires 3 are threaded through two small holes 8 in the carrier member 2 (see fig. 4). Each of the wires 3 are joined to each their conductor (not shown) in a cable 7. At least one of the wires is connected via a resistor 10. The cable 7 is provided in a suitable length and terminated with a plug 11 for connecting the cable 7 to an electrical power source, such as a battery, which the wearer/patient can store in a pocket, attach to the body or the like.

The wire/cable connection, i.e. the section of conductive wires 3 above the carrier member 2, the resistor or resistors 10 and their connection to the cable 7 is arranged in an area 9 and is covered by a protective cover layer 4, which is made of polytetrafluoroethylene (PTFE) or the like. This minimizes expansion of the electrical field in the distal direction, i.e. away from the wearer and hence also directs and thereby increases the electrical field in the direction (the proximal direction) of the wound area, i.e. the damaged tissue. A further protective outer layer 5 is finally adhesively provided to fix the protective cover layer 4 in its position.

In an aspect the base layer 1 is made of a nonwoven medical tape material, such as product no. 1776 supplied from 3M™. The conductive wires 3 are made of filaments of gold, platinum or silver provided in a diameter of 0.15 - 0.40 mm. As mentioned above, the wires 3 are adhesively attached to the underside of the carrier member 2. In the aspect, the carrier member 2 is made of an adhesive sheet product, such as product number 9776 Tan Polyethylene Foam Single Coated Medical Tape from the supplier 3M™. This material is a hypoallergenic pressure sensitive adhesive which is easy to apply and ethylene oxide and gamma compatible in sterilisation and opaque in colour. This material is also used for the protective outer layer 5.

The electric cable 7 is a normal multi-pole cord supplying direct current from a battery or the like. The battery is in the shown aspect a 9 V battery, but dependent on the specific user application, parameters such as size of the plaster, the voltage and the current may be altered. Generally, it is found that the diameter of the filaments forming the conductor wires should be between 0.15-0.40 mm and the voltage should be in the range of 3.5 to 20 volts.

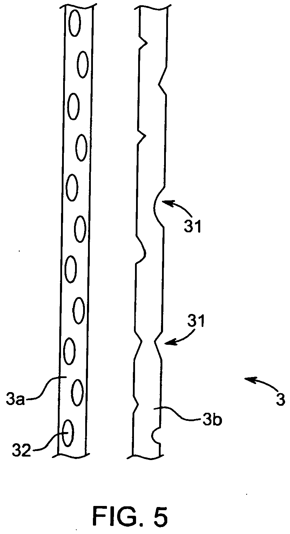

As shown in figures 5 a) and b), the conductor wires or filaments 3a and 3b are pre-treated to be applied an uneven surface structure, e.g. by etching creating depressions 32 in the surface of the filament 3a or by mechanical treatment making indentations 31 in the filament 3b. Other pre-treatments or a combination of pre-treatments may also be used to achieve an uneven surface structure so that the electrical current in the filament becomes as "disturbed" as possible whereby the created electrical field is increased and thereby the electrical exposure of the surroundings.

The objective of the wound treatment achieved by a device which is exemplified in the figures, is that non-linear signals are going down through the tissue among other things hit arteries, arterioles, capillaries, venules and veins. At the capillaries, which are the smallest blood vessels, the exchange of gasses and nutrients and waste matters between the blood and tissue cells takes place. Arteries and arterioles - mentioned according to decreasing size - transport blood from heart to capillaries. Venules and veins - mentioned according to increasing size - return the blood to the heart. When the flow hits these parts, the hormone-like substances nitrogen oxide (NO) and prostacyclin (prostaglandin I₂, PGl₂) are provoked, among other things. NO is inorganic matter which inhibits the adhesion of the thrombocytes. Both are generated in and released from endothelial cells, both cooperate to inhibit the activation of the thrombocytes, and both are relaxing to the musculature of the vessels (vasodilating). This causes blood to flow to the area of the wound.

Nitrogen oxide (NO) is an unstable radical gas which is synthesized from molecular oxygen and L-arginine in a reaction catalysed by the NO synthase (Wilkinson-Berka, 2004)

Arginine + 1½ NADPH + 2O₂ → NO + citrulline + 2H₂O + 1½ NADP⁺

NO takes part in essential physiological processes such as the communication between peripheral nerves and smooth muscles, control of blood pressure (Pieper et al., 1998). The production of NO takes place by a number of different enzymes which are called NO synthases (NOS) depending on in which tissue the synthesis takes place. There are three isoforms; endothelial (eNOS), neuronal (nNOS) and inducing (iNOS) (*Reviewed in* Föstermann *et al*, 1994).

The object of the present invention is to supply a device in form of a plaster which only by means of precious metals and a DC power supply, e.g. in form of a battery, can improve the healing of wounds as the parts form an electrical potential.

The two not-connected conductors 3 can also function by the body fluids which are secreted at and in the wound form different chemical compounds, the most pronounced of which is the formation of chlorine compounds in the electrical potential. Other compounds are also formed with the precious metals, these also take part in the disintegration of bacteria as they can destroy the DNA strands of the cells, this is known from several medicaments used in chemotherapy against cancer, products such as CISPLATIN, CARBOPLATIN OXALIPLATIN.

In connection with the electrical field and precious metals different compounds will also enter into the chemical process, compounds which are also known to be antiinflammatory from the traditional medicine.

The system creates an electrical field above the wound and the surrounding tissue. The electrical field affects both the wound and the healthy tissue by stimulating blood and fluid supply as described above.

The plaster will furthermore increase the overall hygienic surroundings of the wound as the plaster to a certain degree is self-cleansing due to the formation of chlorine just as the energy conversion in the electrolytic process improves the evaporation of fluids in and around the wound.

The plaster can be used by ordinary health staff without any major previous training. The system functions by means of very small electrical voltages and does therefore not involve any risk to the patient or treatment provider. Therefore, the treatment can also take place with a minimum of supervision.

The wound treatment can also take place on an outpatient basis as the size of the components is modest and the system can be designed so that free movement is possible.

The plaster can be arranged to fit the size of the wound as the size of the power supply/battery in relation to the overall system can be taken into account.

The invention is carried out in accordance with the principles that will give a plaster which includes one or more non-interconnected conductors/ wires of precious metal connected to an energy source giving direct current, for example in form of a battery which is powerful enough to generate a running current between these conductors.

The conductors are laid in a pattern to create an irregular stress field across the area to be treated.

The direct current, e.g. in form of a battery, can be joined to the plaster as an integrated part or as a removable part. The direct current can also be located outside the plaster.

Via the stimuli of the stress field nitrogen oxide and prostacyclin of the endothelial cells are produced, these are vasodilating and inhibitory on the adhesion and aggregation of the thrombocytes.

## Claims

1. A device for treatment of a wound by attachment to the skin, comprising a set of electrodes (3) arranged on a carrier member (2) and connected to an electrical power source having a first and a second terminal which are connected to each of the electrodes (3),
the set of electrodes comprises two non-interconnected electrically conducting wires (3a, 3b) arranged in a pattern on said carrier member (2) and **characterised in that** the electrically conducting wires (3a, 3b) are pretraeted to be provided with an irregular surface structure to create an irregular stress field above the wound.

2. A device according to claim 1, wherein the conducting wires (3a, 3b) are made of a precious metal, such as gold, silver or platinum.

3. A device according to claim 1 or 2, wherein the conducting wires (3a, 3b) are between 0.15-0.40 mm in diameter.

4. A device according to any of the preceding claims, wherein the set of non-interconnected conducting wires (3a, 3b) are substantially parallelly arranged in a serpentine-like pattern.

5. A device according to any of the preceding claims, wherein the carrier member (2) has a distal side and a proximal side, and the set of conducting wires (3a, 3b) are adhesively attached in the pattern to the proximal side of the carrier member (2) and with a cover sheet (4).

6. A device according to claim 5, wherein the conducting wires (3a, 3b) penetrate through the carrier member (2) where the wires (3a, 3b) are connected on the distal side of the carrier member (2) to a cable (7) from the power supply.

7. A device according to claim 5 or 6, wherein at least one wire (3a, 3b) is connected to the cable (7) via a resistor (10), preferably having an electrical resistance of approx. 1 kΩ.

8. A device according to any of claims 5 to 7, wherein said connection between the wires (3a, 3b) and the cable (7) is covered with an electrically shielding sheet material (5) and a covering top sheet thereon.

9. A device according to any of the preceding claims, wherein the electrical power source provides a direct current, preferably with a voltage between 3.5-20 V with a current preferably less than 400 mA.

10. A device according to any of the preceding claims, wherein at least one of the non-interconnected conducting wires (3a, 3b) is pre-treated mechanically so that the physical structure of the wire is altered in several random places.

11. A device according to any of the preceding claims, wherein at least one of the non-interconnected conducting wires (3a, 3b) is pre-treated with a chemical treatment of the at least one wire whereby material can both be removed and another conductive material can be added to the surface of the wire.

12. A device according to any of the preceding claims, wherein at least one of the non-interconnected conducting wires (3a, 3b) is pre-treated with an electrolytic treatment, whereby a corrosion in the surface allowing for absorbing a pharmaceutical product, such as a medicine, which is added to the wire for being deposited in the tissue.

## Patentansprüche

1. Vorrichtung zur Behandlung einer Wunde mittels Befestigung derselben an der Haut, umfassend einen Satz an Elektroden (3), die an einem Trägerelement (2) angeordnet und an einer elektrischen Stromquelle angeschlossen sind, mit einem ersten und einem zweiten Terminal, die jeweils an jeder der Elektroden (3) angeschlossen sind, wobei der Satz an Elektroden zwei nicht miteinander angeschlossene, elektrisch leitende Drähte (3a, 3b) umfasst, die in einem Muster am Trägerelement (2) angeordnet sind und **dadurch gekennzeichnet sind, dass** die elektrisch leitenden Drähte (3a, 3b) bevorzugt mit einer unregelmäßigen Oberflächenstruktur bereitzustellen sind, um über der Wunde ein unregelmäßiges Stressfeld zu erzeugen.

2. Vorrichtung nach Anspruch 1, wobei die leitenden Drähte (3a, 3b) aus Edelmetall bestehen, wie z.B. Gold, Silber oder Platin.

3. Vorrichtung nach Anspruch 1 oder 2, wobei die leitenden Drähte (3a, 3b) einen Durchmesser von zwischen 0,15 - 0,40 mm aufweisen.

4. Vorrichtung nach einem der vorstehenden Ansprüche, wobei der Satz an nicht miteinander angeschlossenen, leitenden Drähten (3a, 3b) im Wesentlichen in einem schlangenförmigen Muster parallel angeordnet ist.

5. Vorrichtung nach einem der vorstehenden Ansprüche, wobei das Trägerelement (2) eine distale Seite und eine proximale Seite aufweist und der Satz an leitenden Drähten (3a, 3b) mittels Verkleben im Muster an der proximalen Seite des Trägerelements (2) befestigt ist und eine Abdeckung (4) aufweist.

6. Vorrichtung nach Anspruch 5, wobei die leitenden Drähte (3a, 3b) das Trägerelement (2) an der Stelle durchdringen, an welcher die Drähte (3a, 3b) an der distalen Seite des Trägerelements (2) an einem Kabel (7) der Stromquelle angeschlossen sind.

7. Vorrichtung nach Anspruch 5 oder 6, wobei wenigstens ein Draht (3a, 3b) über einen Widerstand (10) am Kabel (7) angeschlossen ist, bevorzugt mit einem elektrischen Widerstand von etwa 1 kΩ.

8. Vorrichtung nach einem der Ansprüche 5 bis 7, wobei der Anschluss zwischen den Drähten (3a, 3b) und dem Kabel (7) mit einem elektrisch abschirmenden Abdeckungsmaterial (5) und einer Abdeckungsoberseite darauf bedeckt ist.

9. Vorrichtung nach einem der vorstehenden Ansprüche, wobei die elektrische Stromquelle einen Gleichstrom bereitstellt, bevorzugt mit einer Spannung von zwischen 3, 5 - 20 V, mit einem Strom von bevorzugt höchstens 400 mA.

10. Vorrichtung nach einem der vorstehenden Ansprüche, wobei wenigstens einer der nicht miteinander angeschlossenen, leitenden Drähte (3a, 3b) mechanisch vorbehandelt ist, sodass die physikalische Struktur des Drahts an mehreren wahllosen Stellen verändert wird.

11. Vorrichtung nach einem der vorstehenden Ansprüche, wobei wenigstens einer der nicht miteinander angeschlossenen, leitenden Drähte (3a, 3b) mit einer chemischen Behandlung des wenigstens einen Drahts vorbehandelt ist, wobei das Material sowohl entfernt als auch ein weiteres leitendes Material zur Oberfläche des Drahts hinzugefügt werden kann.

12. Vorrichtung nach einem der vorstehenden Ansprüche, wobei wenigstens einer der nicht miteinander angeschlossenen, leitenden Drähte (3a, 3b) mit einer elektrolytischen Behandlung vorbehandelt ist, wobei eine Korrosion in der Oberfläche die Absorption eines pharmazeutischen Produkts erlaubt, wie z.B. eine Medizin, die zum Draht hinzugefügt wird, der im Gewebe eingelegt werden soll.

## Revendications

1. Dispositif de traitement d'une plaie par fixation sur la peau, comprenant un ensemble d'électrodes (3) disposé sur un élément de support (2) et relié à une source d'alimentation électrique ayant une première et une deuxième borne qui sont reliées à chacune des électrodes (3) ,
l'ensemble d'électrodes comprend deux fils conducteurs électriquement non interconnectés (3a, 3b) disposés selon un motif sur ledit élément de support (2) et est **caractérisé en ce que** les fils électriquement conducteurs (3a, 3b) sont prétraités pour être dotés d'une structure de surface irrégulière pour créer un champ de contrainte irrégulier au-dessus de la plaie.

2. Dispositif selon la revendication 1, dans lequel les fils conducteurs (3a, 3b) sont faits d'un métal précieux, tel que or, argent ou platine.

3. Dispositif selon la revendication 1 ou 2, dans lequel les fils conducteurs (3a, 3b) font entre 0,15 et 0,40 mm de diamètre.

4. Dispositif selon l'une quelconque des revendications précédentes, dans lequel l'ensemble de fils conducteurs non interconnectés (3a, 3b) sont disposés sensiblement en parallèle dans un motif ressemblant à un serpentin.

5. Dispositif selon l'une quelconque des revendications précédentes, dans lequel l'élément de support (2) a un côté distal et un côté proximal, et l'ensemble de fils conducteurs (3a, 3b) est fixé par un adhésif dans le motif à la face proximale de l'élément de support (2) et avec une feuille de couverture (4).

6. Dispositif selon la revendication 5, dans lequel les fils conducteurs (3a, 3b) pénètrent à travers l'élément de support (2) où les fils (3a, 3b) sont reliés sur le côté distal de l'élément de support (2) à un câble (7) à partir du bloc d'alimentation.

7. Dispositif selon la revendication 5 ou 6, dans lequel au moins un fil métallique (3a, 3b) est relié au câble (7) par l'intermédiaire d'une résistance (10), ayant de préférence une résistance électrique d'environ 1 kΩ.

8. Dispositif selon l'une quelconque des revendications 5 à 7, dans lequel ladite connexion entre les fils (3a, 3b) et le câble (7) est recouverte d'un matériau en feuille protégeant électriquement (5) et d'une feuille supérieure de couverture sur celle-ci.

9. Dispositif selon l'une quelconque des revendications précédentes, dans lequel la source d'alimentation électrique fournit un courant continu, de préférence avec une tension comprise entre 3,5 à 20 V avec un courant de préférence inférieur à 400 mA.

10. Dispositif selon l'une quelconque des revendications précédentes, dans lequel au moins l'un des fils conducteurs non interconnectés (3a, 3b) est prétraité mécaniquement de sorte que la structure physique du fil soit modifiée en plusieurs endroits aléatoires.

11. Dispositif selon l'une quelconque des revendications précédentes, dans lequel au moins un des fils conducteurs non interconnectés (3a, 3b) est prétraité avec un traitement chimique d'au moins un fil de sorte que à la fois du matériau puisse être retiré et un autre matériau conducteur puisse être ajouté à la surface du fil.

12. Dispositif selon l'une quelconque des revendications précédentes, dans lequel au moins l'un des fils conducteurs non interconnectés (3a, 3b) est prétraité avec un traitement électrolytique, grâce à quoi une corrosion de la surface permet l'absorption d'un produit pharmaceutique, tel qu'un médicament, qui est ajouté au fil pour être déposé dans les tissus.
